# EUROPEAN PATENT APPLICATION

(11) **EP 1 375 640 A1**
(43) Date of publication of application: **02.01.2004**
(21) Application number: 03253781.3
(22) Date of filing: 16.06.2003
(51) Int. Cl.: C12N 1/16, A61K 35/66, A23K 1/18, A23K 1/00, C12N 13/00

(54) **Feed additives for cattle: prevention of E.Coli infection**

(30) Priority: 18.06.2002 US 175015
(71) Applicant: Ultra Biotech Limited, Douglas IM1 1SA, Isle of Man (GB)
(72) Inventor: Cheung, Ling Yuk, New Territories (HK)
(74) Representative: Taylor, Kathryn May

(57) **Abstract**

The present invention relates to feed additives for prevention of E. coli infection in cattle. The invention provides methods for making a biological compositions comprising yeast cells that can improve the immune functions of cattle such that the incidence of E. coli infection is reduced. The invention also relates to methods for manufacturing the biological compositions, and methods of using the biological compositions as feed additives.

## Description

### 1. FIELD OF THE INVENTION

The invention relates to biological compositions comprising yeast cells that can improve the immune functions of animals. The invention also relates to methods for manufacturing the biological compositions, and methods of using the biological compositions as animal feed additives.

### 2. BACKGROUND OF THE INVENTION

*Escherichia coli* serotype O157:H was only recognized as a human pathogen a little more than a decade ago, yet it has become a major foodborne pathogen. In the United States, the severity of serotype O157:H7 infections in the young and the elderly has had a tremendous impact on human health, the food industry, and federal regulations regarding food safety. In the USA, the Centers for Disease Control estimates that 73,000 cases of infection and 61 deaths in the United States can be attributed to *E. coli* 0157:H7 annually. Most infections have been from consumption of contaminated juice, meat and other foods.

Enterohemorrhagic *Escherichia coli* (EHEC) serotype O157:H7 has emerged in recent years as the predominant cause of hemorrhagic colitis in humans. This illness, with characteristic symptoms of bloody diarrhea and abdominal cramps, can progress into a more severe, life-threatening complication known as hemolytic uremic syndrome (HUS). It can cause kidney failure and death, primarily in children and immune compromised adults. The pathogenicity of EHEC appears to be associated with a number of virulence factors, including the production of toxins collectively referred to as verotoxins or Shiga-like toxins. Epidemiologic investigations of past outbreaks showed that most have been associated with the consumption of bovine products. A small percentage of cattle are carriers of *E. coli* 0257:H7. When meat is contaminated with cattle feces at slaughter, this strain of *E. coli* can enter the food chain. Reducing the levels of *E. coli* O157:H7 organisms that enter slaughter plants would require two strategies: (i) reducing the number of cattle shedding *E. coli* 0157:H7 and (ii) reducing the magnitude of shedding by those animals infected with the organism (Cray Jr., et al., Applied Environmental Microbiology: May 1998, p.1975-1979, Vol. 64, No. 5).

Dietary management during the preslaughter period of beef production may thus play a role in reducing the incidence of *E. coli* 0157:H7-positive ruminants. There has been conflicting information on the effect of diet on *E. coli* shedding from cattle. The conflict arises in part from the effect of diet on the ability of *E. coli* to develop acid resistance. Acid resistant bacteria are able to survive stomach acid in humans, reproduce, and produce the toxins that cause disease (Diez-Gonzalez et al., Science: Sept 11, 1998. Volume 281, Number 5383, pages 1666-1668.) However, Hancock et al. (Science, April 2, 1999 Volume 284, Number 5411, page 49) contend that this conclusion is unsupported or contradicted by several lines of evidence. The *E. coli* that contaminate beef typically originate from the hide, the hooves, or the equipment used in slaughter and processing rather than directly from the colon, and likely replicate in environments unlike the colon. Many in the food and agriculture industry believe that there is a need for further study of cattle feeding management practices and its use in decreasing the risk of foodborne illness from *E. coli.* The present invention provides a solution that uses yeasts to improve the immune functions of cattle and hence reduce the number of cattle shedding *E. coli* O157:H7

The inclusion of fungal cells or fungal fermentation products in animal feed have been in use for some time. Certain bacteria, yeasts and mold preparations, commonly referred to as probiotics or direct fed microbials, are administered orally or added to animal feeds to provide various benefits. However, the mechanism of action of such preparations are not properly understood but it is believed that they act by altering the gut microflora/microbiota of the animals thereby improving the health of the intestinal tract lining and allowing for improved nutrient absorption. In the case of ruminants, the preparation may ameliorate fermentation in the rumen that results in the wasteful production of gases.

For examples of the use of fungal cells and products in animal feed, see the annual Feed Additive Compendium published by The Miller Publishing Company (Minnetonka, Minn.) or the following patent literature:
United States Patent No. 3,903,307 discloses a process for making animal feed that is based on the fermentation of waste molasses or bagasse by yeasts, such as *Candida utilis* and *Trichoderma veride.*
U.S. Patent No. 4,055,667 discloses a liquid animal feed supplement that comprises a colloidal mixture of spent brewer's yeast in an aqueous alcoholic medium.
U.S. Patent No. 4,582,708 discloses an animal feed supplement that comprises live yeast cells *(Saccharomyces* species) having fermenting activity, a texturizing component comprising ground meal, ground legumes or mixtures thereof, a mineral mixture, a liquid binder, a vitamin mixture, and ground montmorillonite.
U.S. Patent No. 5,624,686 discloses an animal feed additive that is prepared by cultivating certain bacteria or yeast species (such as *Saccharomyces cerevisiae, Candida utilis)* and disrupting the microbial cells such that metabolites of the cells are efficiently made available to the animal.
U.S. Patent No. 6,214,337 discloses an animal feed that comprises yeast glucan which is derived from the cell walls of various yeast species (such as *Saccharomyces cerevisiae, Candida utilis).*
Citation of documents herein is not intended as an admission that any of the documents cited herein is pertinent prior art, or an admission that the cited documents are considered material to the patentability of the claims of the present application. All statements as to the date or representations as to the contents of these documents are based on the information available to the applicant and does not constitute any admission as to the correctness of the dates or contents of these documents.

### 3. SUMMARY OF THE INVENTION

The present invention relates to biological compositions that can be added to animal feed to reduce the infection of large ruminants, such as cattle, by *E. coli.*

In one embodiment, the present invention provides biological compositions comprising a plurality of live yeast cells which are capable of improving the immune functions of large ruminants, such as cattle, when ingested. The biological compositions can be used for reducing infections in cattle by pathogenic strains of *Escherichia coli.*

In another embodiment, the invention provides methods of making the biological composition. In particular, the methods of the invention comprise culturing yeast cells in the presence of a series of electromagnetic fields of defined frequencies and field strengths, such that the yeast cells becomes metabolically active and potent at stimulating an animal's immune system. Up to four different components of yeast cells can be used to form the biological compositions. The yeast cells can also be subjected to a conditioning or acclimatizing step to improve its performance. The conditioning step comprises culturing the yeast cells in a culture medium comprising gastric juice of an animal and wild hawthorn juice. Methods for manufacturing the biological compositions comprising culturing the yeast cells under activation conditions, mixing various yeast cell cultures of the present invention, followed by drying the yeast cells and packing the final product, are encompassed. In preferred embodiments, the starting yeast cells are commercially available and/or accessible to the public, such as but not limited to *Saccharomyces cerevisiae.*

The biological compositions of the invention can be fed directly to animals or used as an additive to be incorporated into regular animal feed. Animal feed compositions comprising activated yeast cells of the invention and ingredients such as zeolite powder are encompassed by the invention.

### 4. BRIEF DESCRIPTION OF FIGURES

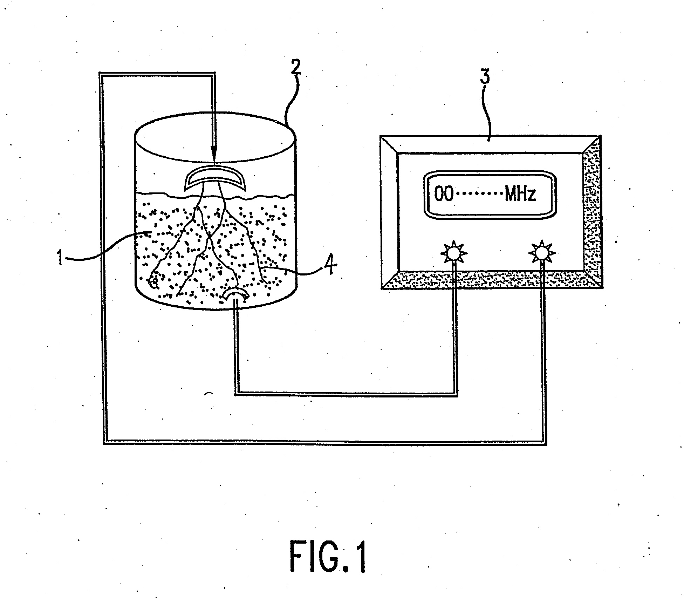

Fig. 1 Activation and conditioning of yeast cells. 1 yeast cell culture; 2 container; 3 electromagnetic field source; 4 electrode.

### 5. DETAILED DESCRIPTION OF THE INVENTION

The present invention relates to biological compositions that can improve the immune functions of animals, and/or reduce the incidence of infections by pathogenic strains of *E. coli.* The present invention provides methods for manufacturing the biological compositions as well as methods for using the biological compositions as animal feed additives.

The biological compositions of the invention comprise yeasts. Unlike the traditional use of yeasts as a component of the feed, the yeast cells of the invention are not a primary source of nutrients for the animals. The yeast cells of the invention serve as a supplement to replace or reduce the antibiotics that are now routinely added to livestock feed. The yeast cells are live when administered orally or ingested along with feed by the animals. While in the gastrointestinal tract of an animal, the yeast cells are capable of stimulating the immune system and improving the immune functions of the animal, thereby reducing the incidence of infectious diseases, especially infections by pathogenic strains of *E. coli.* The use of the biological compositions of the invention can lower the overall cost of maintaining the health of animals in commercial animal operations, and reduce the contamination of meat products by pathogenic strains of *E. coli.*

While the following terms are believed to have well-defined meanings in the art, the following are set forth to define the terms as used herein, and facilitate explanation of the invention.

As used herein, the term "feed" broadly refers to any kind of material, liquid or solid, that is used for nourishing an animal, and for sustaining normal or accelerated growth of an animal including newborns and young developing animals. Preferably, the feed is cattle feed.

The term "animal" as used herein refers to cattle or domestic cow *(Bos taurus)* generally, and also includes but not limited to dairy cows, beef cattle, bisons, and buffalos.

The term "immune functions" as used herein broadly encompasses specific and non-specific immunological reactions of the animal, and includes both humoral and cell-mediated defense mechanisms. The immune functions of the animal enable the animal to survive and/or recover from an infection by a pathogen, such as bacteria, viruses, fungi, protozoa, helminths, and other parasites. The immune functions of the animal can also prevent infections, particularly future infections by the same pathogen after the animal had an initial exposure to the pathogen. Many types of immune cells are involved in providing the immune functions, which include various subsets of lymphocytes (B cells, T cells, K cells, NK cells), different types of leukocytes (macrophages, neutrophils, eosinophils, basophils), antigen presenting cells (dendritic cells, endothelial cells) and cells that are found in specialized organs and tissues with immunological activities (bone marrow, lymph nodes, thymus, bursa, Peyer's patch). Details of the immune system of ruminants are described in The Ruminant Immune System by John E. Butler, 1981, Perseus Books, which is incorporated herein by reference in its entirety.

In one embodiment, the present invention provides biological compositions that comprise at least one yeast cell component. Each yeast cell component comprises a population of live yeast cells which have been cultured under a specific set of conditions such that the yeast cells are capable of improving the immune functions of an animal. In preferred embodiments, the biological compositions of the invention comprise up to four yeast cell components. The yeasts of the invention are capable of reducing the incidence of infection by pathogenic *E. coli* including suppressing their growth and proliferation in culture.

According to the invention, under certain specific culture conditions, yeasts can be made metabolically active such that they become effective in stimulating and enhancing the immune functions of an animal which ingested the yeasts. Without being bound by any theory or mechanism, the inventor believes that the culture conditions activate and/or amplified the expression of a gene or a set of genes in the yeast cells such that the yeast cells metabolize at a high rate, and also becomes highly potent in stimulating the animal's immune system. It is envisioned that interactions between certain yeast gene products and pathogenic organisms and/or elements of the animal's immune system is greatly enhanced by the elevated levels of these yeast gene products after the yeast cells have been cultured under the conditions described hereinbelow. Interactions with the immune system are believed to involve immune cells lining the gastrointestinal tract, lymph nodes, as well as circulating immune cells. As a result of these interactions, the immune functions of the animals, such as responsiveness to and recovery from an infection, and resistance to diseases, are improved. The animals are thus less prone to infection by many pathogenic strains of *E. coli.* The presence of the metabolically activated yeast cells are also thought to compete with and deprive pathogenic organisms of essential nutrients and growth factors.

As used herein, the term "pathogenic *E. coli"* encompasses any strain of *E. coli* that causes a disease symptom in humans or that produces a verotoxin or Shiga-like toxin. The term includes but is not limited to enterohemorrhagic *E*. *coli* strains and isolate O157:H7 in particular. The clonal nature of serotype 0157:H7 has facilitated its phenotypic identification. Unlike other *E*. *coli,* isolates of serotype O157:H7 do not ferment sorbitol in 24 hours and are negative in the methyl-umbelliferyl glucuronide assay, which measures glucuronidase activity. These phenotypes, especially the absence of sorbitol fermentation, are used extensively to distinguish isolates of serotype 0157:H7 from related bacteria. Isolation of serotype O157:H from foods, on selective media, such as hemorrhagic colitis agar and cefixime-tellurite sorbitol-MacConkey agar is based on the sorbitol phenotype.

In one embodiment, the biological compositions of the invention can be fed directly to an animal. In another embodiment, the biological compositions can be added to the feed. As known to those skilled in the relevant art, many methods and appliances may be used to mix the biological compositions of the invention with feed. In a particular embodiment, a mixture of culture broths of the yeasts of the present invention are added directly to the feed just prior to feeding the animal. Dried powders of the yeasts can also be added directly to the feed just prior to feeding the animal. In yet another embodiment of the present invention, the yeast cells are mixed with the raw constituents of the feed with which the yeast cells become physically incorporated. The biological compositions may be applied to and/or mixed with the feed by any mechanized means which may be automated.

The amount of biological composition used depends in part on the feeding regimen and the type of feed, and can be determined empirically. For example, the useful ratio of biological composition to animal feed ranges from 0.1% to 1% by dry weight, preferably, 0.3 to 0.8%, and most preferably at about 0.5%. Although not necessary, the biological compositions of the invention can also be used in conjunction or in rotation with other types of supplements, such as but not limited to vitamins, minerals, and vaccines.

Described below in Section 5.1 and 5.2 are four yeast cell components of the invention and methods of their preparation. Section 5.3 describes the manufacture of the biological compositions of the invention which comprises at least one of the four yeast cell components.

### 5.1. PREPARATION OF THE YEAST CELL CULTURES

The present invention provides yeast cells that are capable of improving the immune functions of an animal which ingested the yeast cells. Up to four different yeast cell components can be combined to make the biological compositions.

A yeast cell component of the biological composition is produced by culturing a plurality of yeast cells in an appropriate culture medium in the presence of an alternating electromagnetic field or multiple alternating electromagnetic fields in series over a period of time. The culturing process allows yeast spores to germinate, yeast cells to grow and divide, and can be performed as a batch process or a continuous process. As used herein, the terms "alternating electromagnetic field", "electromagnetic field" or "EM field" are synonymous. An electromagnetic field useful in the invention can be generated by various means well known in the art. A schematic illustration of exemplary setups are depicted respectively in Fig. 1. An electromagnetic field of a desired frequency and a desired field strength is generated by an electromagnetic wave source (3) which comprises one or more signal generators that are capable of generating electromagnetic waves, preferably sinusoidal waves, and preferably in the frequency range of 1500 MHz - 15000 MHz. Such signal generators are well known in the art. Signal generators capable of generating signal with a narrower frequency range can also be used. If desirable, a signal amplifier can also be used to increase the output signal, and thus the strength of the EM field.

The electromagnetic field can be applied to the culture by a variety of means including placing the yeast cells in close proximity to a signal emitter connected to a source of electromagnetic waves. In one embodiment, the electromagnetic field is applied by signal emitters in the form of electrodes that are submerged in a culture of yeast cells (1). In a preferred embodiment, one of the electrodes is a metal plate which is placed on the bottom of a non-conducting container (2), and the other electrode comprises a plurality of wires or tubes so configured inside the container such that the energy of the electromagnetic field can be evenly distributed in the culture. For an upright culture vessel, the tips of the wires or tubes are placed within 3 to 30 cm from the bottom of the vessel (i.e, approximately 2 to 10% of the height of the vessel from the bottom). The number of electrode wires used depends on both the volume of the culture and the diameter of the wire. For example, for a culture having a volume of 10 liter or less, two or three electrode wires having a diameter of between 0.5 to 2.0 mm can be used. For a culture volume of 10 liter to 100 liter of culture, the electrode wires or tubes can have a diameter of 3.0 to 5.0 mm. For a culture volume of 100 liter to 1000 liter, the electrode wires or tubes can have a diameter of 6.0 to 15.0 mm. For a culture having a volume greater than 1000 liter, the electrode wires or tubes can have a diameter of between 20.0 to 25.0 mm.

In various embodiments, yeasts of the genera of *Saccharomyces, Candida, Crebrothecium, Geotrichum, Hansenula, Kloeckera, Lipomyces, Pichia, Rhodosporidium, Rhodotorula Torulopsis, Trichosporon,* and *Wickerhamia* can be used in the invention.

Non-limiting examples of yeast strains include *Saccharomyces cerevisiae* Hansen, ACCC2034, ACCC2035, ACCC2036, ACCC2037, ACCC2038, ACCC2039, ACCC2040, ACCC2041, ACCC2042, AS2.1, AS2.4, AS2.11, AS2.14, AS2.16, AS2.56, AS2.69, AS2.70, AS2.93, AS2.98, AS2.101, AS2.109, AS2.110, AS2.112, AS2.139, AS2.173, AS2.174, AS2.182, AS2.196, AS2.242, AS2.336, AS2.346, AS2.369, AS2.374, AS2.375, AS2.379, AS2.380, AS2.382, AS2.390, AS2.393, AS2.395, AS2.396, AS2.397, AS2.398, AS2.399, AS2.400, AS2.406, AS2.408, AS2.409, AS2.413, AS2.414, AS2.415, AS2.416, AS2.422, AS2.423, AS2.430, AS2.431, AS2.432, AS2.451, AS2.452, AS2.453, AS2.458, AS2.460, AS2.463, AS2.467, AS2.486, AS2.501, AS2.502, AS2.503, AS2.504, AS2.516, AS2.535, AS2.536, AS2.558, AS2.560, AS2.561, AS2.562, AS2.576, AS2.593, AS2.594, AS2.614, AS2.620, AS2.628, AS2.631, AS2.666, AS2.982, AS2.1190, AS2.1364, AS2.1396, IFFI 1001, IFFI 1002, IFFI 1005, IFFI 1006, IFFI 1008, IFFI 1009, IFFI 1010, IFFI 1012, IFFI 1021, IFFI 1027, IFFI 1037, IFFI 1042, IFFI 1043, IFFI 1045, IFFI 1048, IFFI 1049, IFFI 1050, IFFI 1052, IFFI 1059, IFFI 1060, IFFI 1063, IFFI 1202, IFFI 1203, IFFI 1206, IFFI 1209, IFFI 1210, IFFI 1211, IFFI 1212, IFFI 1213, IFFI 1215, IFFI 1220, IFFI 1221, IFFI 1224, IFFI 1247, IFFI 1248, IFFI 1251, IFFI 1270, IFFI 1277, IFFI 1287, IFFI 1289, IFFI 1290, IFFI 1291, IFFI 1292, IFFI 1293, IFFI 1297, IFFI 1300, IFFI 1301, IFFI 1302, IFFI 1307, IFFI 1308, IFFI 1309, IFFI 1310, IFFI 1311, IFFI 1331, IFFI 1335, IFFI 1336, IFFI 1337, IFFI 1338, IFFI 1339, IFFI 1340, IFFI 1345, IFFI 1348, IFFI 1396, IFFI 1397, IFFI 1399, IFFI 1411, IFFI *1413; Saccharomyces cerevisiae* Hansen Var. ellipsoideus (Hansen) Dekker, ACCC2043, AS2.2, AS2.3, AS2.8, AS2.53, AS2.163, AS2.168, AS2.483, AS2.541, AS2.559, AS2.606, AS2.607, AS2.611, AS2.612; *Saccharomyces chevalieri* Guillermond, AS2.131, AS2.213; *Saccharomyces delbrueckii,* AS2.285; *Saccharomyces delbrueckii* Lindner var. mongolicus Lodder et van Rij, AS2.209, AS2.1157; *Saccharomyces exiguous* Hansen, AS2.349, AS2.1158; *Saccharomyces fermentati* (Saito) Lodder et van Rij, AS2.286, AS2.343; *Saccharomyces logos* van laer et Denamur ex Jorgensen, AS2.156, AS2.327, AS2.335; *Saccharomyces mellis* Lodder et Kreger Van Rij, AS2.195; *Saccharomyces microellipsoides* Osterwalder, AS2.699; Saccharomyces *oviformis* Osterwalder, AS2.100; *Saccharomyces rosei* (Guilliermond) Lodder et kreger van Rij, AS2.287; *Saccharomyces rouxii* Boutroux, AS2.178, AS2.180, AS2.370, AS2.371; *Saccharomyces sake* Yabe, ACCC2045; *Candida arborea,* AS2.566; *Candida Krusei* (Castellani) Berkhout, AS2.1045; *Candida lambica(Lindner* et Genoud) van.Uden et Buckley, AS2.1182; *Candida lipolytica* (Harrison) Diddens et Lodder, AS2.1207, AS2.1216, AS2.1220, AS2.1379, AS2.1398, AS2.1399, AS2.1400; *Candida parapsilosis* (Ashford) Langeron et Talice, AS2.590; *Candida parapsilosis* (Ashford) et Talice Var. intermedia Van Rij et Verona, AS2.491; *Candida pulcherriman* (Lindner) Windisch, AS2.492; *Candida rugousa* (Anderson) Diddens et Loddeer, AS2.511, AS2.1367, AS2.1369, AS2.1372, AS2.1373, AS2.1377, AS2.1378, AS2.1384; *Candida tropicalis* (Castellani) Berkout, ACCC2004, ACCC2005, ACCC2006, AS2.164, AS2.402, AS2.564, AS2.565, AS2.567, AS2.568, AS2.617, AS2.1387; *Candida utilis* Henneberg Lodder et Kreger Van Rij, AS2.120, AS2.281, AS2.1180; *Crebrothecium ashbyii* (Guillermond) Routein, AS2.481, AS2.482, AS2.1197; *Geotrichum candidum* Link,. ACCC2016, AS2.361, AS2.498, AS2.616, AS2.1035, AS2.1062, AS2.1080, AS2.1132, AS2.1175, AS2.1183; *Hansenula anomala* (Hansen) H et P sydow, ACCC2018, AS2.294, AS2.295, AS2.296, AS2.297, AS2.298, AS2.299, AS2.300, AS2.302, AS2.338, AS2.339, AS2.340, AS2.341, AS2.470, AS2.592, AS2.641, AS2.642, AS2.635, AS2.782, AS2.794; *Hansenula arabitolgens* Fang, AS2.887; *Hansenula jadinii* Wickerham, ACCC2019; *Hansenula saturnus* (Klocker) H et P sydow, ACCC2020; *Hansenula schneggii* (Weber) Dekker, AS2.304; *Hansenula subpelliculosa* Bedford, AS2.738, AS2.740, AS2.760, AS2.761, AS2.770, AS2.783, AS2.790, AS2.798, AS2.866; *Kloeckera apiculata* (Reess emend. Klocker) Janke, ACCC2021, ACCC2022, ACCC2023, AS2.197, AS2.496, AS2.711, AS2.714; *Lipomyces starkeyi* Lodder et van Rij, ACCC2024, AS2.1390; *Pichia farinosa* (Lindner) Hansen, ACCC2025, ACCC2026, AS2.86, AS2.87, AS2.705, AS2.803; *Pichia membranaefaciens* Hansen, ACCC2027, AS2.89, AS2.661, AS2.1039; *Rhodosporidium toruloides* Banrio, ACCC2028; *Rhodotorula glutinis* (Fresenius) Harrison, ACCC2029, AS2.280, ACCC2030, AS2.102, AS2.107, AS2.278, AS2.499, AS2.694, AS2.703, AS2.704, AS2.1146*; Rhodotorula minuta* (Saito) Harrison, AS2.277; *Rhodotorula rubar* (Demme) Lodder, ACCC2031, AS2.21, AS2.22, AS2.103, AS2.105, AS2.108, AS2.140, AS2.166, AS2.167, AS2.272, AS2.279, AS2.282; *Saccharomyces carlsbergensis* Hansen, AS2.113, ACCC2032, ACCC2033, AS2.312, AS2.116, AS2.118, AS2.121, AS2.132, AS2.162, AS2.189, AS2.200, AS2.216, AS2.265, AS2.377, AS2.417, AS2.420, AS2.440, AS2.441, AS2.443, AS2.444, AS2.459, AS2.595, AS2.605, AS2.638, AS2.742, AS2.745, AS2.748, AS2.1042; *Saccharomyces uvarum Beijer*, IFFI 1023, IFFI 1032, IFFI 1036, IFFI 1044, IFFI 1072, IFFI 1205, IFFI 1207; *Saccharomyces willianus* Saccardo, AS2.5, AS2.7, AS2.119, AS2.152, AS2.293, AS2.381, AS2.392, AS2.434, AS2.614, AS2.1189; *Saccharomyces sp.,* AS2.311; *Saccharomyces ludwigii* Hansen, ACCC2044, AS2.243, AS2.508; *Saccharomyces sinenses* Yue, AS2.1395; *Schizosaccharomyces octosporus* Beijerinck, ACCC 2046, AS2.1148; *Schizosaccharomyces pombe* Linder, ACCC2047, ACCC2048, AS2.248, AS2.249, AS2.255, AS2.257, AS2.259, AS2.260, AS2.274, AS2.994, AS2.1043, AS2.1149, AS2.1178, IFFI 1056; *Sporobolomyces roseus* Kluyver et van Niel, ACCC 2049, ACCC 2050, AS2.619, AS2.962, AS2.1036, ACCC2051, AS2.261, AS2.262; *Torulopsis candida* (Saito) Lodder, ACCC2052, AS2.270; *Torulopsis famta* (Harrison) Lodder et van Rij, ACCC2053, AS2.685; *Torulopsis globosa* (Olson et Hammer) Lodder et van Rij, ACCC2054, AS2.202; *Torulopsis inconspicua* Lodder et van Rij, AS2.75; *Trichosporon behrendii* Lodder et Kreger van Rij, ACCC2055, AS2.1193; *Trichosporon capitatum* Diddens et Lodder, ACCC2056, AS2.1385; *Trichosporon cutaneum(de* Beurm et al.)Ota, ACCC2057, AS2.25, AS2.570, AS2.571, AS2.1374; *Wickerhamia fluoresens* (Soneda) Soneda, ACCC2058, AS2.1388. Yeasts of the *Saccharomyces* genus are generally preferred. *Saccharomyces cerevisiae* and *Saccharomyces carlsbergensis* are preferred strains.

Generally, yeast strains useful for the invention can be obtained from private or public laboratory cultures, or publically accessible culture deposits, such as the American Type Culture Collection, 10801 University Boulevard, Manassas, VA 20110-2209 and the China General Microbiological Culture Collection Center (CGMCC), China Committee for Culture Collection of Microorganisms, Institute of Microbiology, Chinese Academy of Sciences, Haidian, P.O. Box 2714, Beijing, 100080, China.

Although it is preferred, the preparation of the yeast cell components of the invention is not limited to starting with a pure strain of yeast. Each yeast cell component may be produced by culturing a mixture of yeast cells of different species or strains. The constituents of a yeast cell component can be determined by standard yeast identification techniques well known in the art.

In various embodiments of the invention, standard techniques for handling; transferring, and storing yeasts are used. Although it is not necessary, sterile conditions or clean environments are desirable when carrying out the manufacturing processes of the invention. Standard techniques for handling animal blood and immune cells, and for studying immune functions of an animal are also used. Details of such techniques are described in Advances in Laboratory Methods: General Haematology, 2000, Assendelft et al., (Ed.), Arnold, Edward (Publisher); Handbook of Vertebrate Immunology, 1998, Pastoret et al. (Ed.), Academic Press, and Current Protocols In Immunology, 1991, Coligan, et al. (Ed), John Wiley & Sons, Inc., which are both incorporated herein by reference in their entireties.

In one embodiment, the yeast cells of the first yeast cell component are cultured in the presence of at least one alternating electromagnetic (EM) field with a frequency in the range of 7700 MHz to 7730 MHz, preferably 7700 to 7722 MHz. A single EM field or a series of EM fields can be applied, each having a different frequency within the stated range, or a different field strength within the stated range, or different frequency and field strength within the stated ranges. Although any practical number of EM fields can be used within a series, it is preferred that, the yeast culture be exposed to a total of 2, 3, 4, 5, 6, 7, 8, 9 or 10 different EM fields in a series. The EM field(s), which can be applied by any means known in the art, can each have a frequency of 7700, 7701, 7702, 7703, 7704, 7705, 7706, 7707, 7708, 7709, 7710, 7711, 7712, 7713, 7714, 7715, 7716, 7717, 7718, 7719, 7720, 7721 or 7722 MHz. The field strength of the EM field(s) is in the range of 20 to 220 mV/cm preferably 156 ± 2.0 mV/cm. The yeast cells can be cultured in the EM fields for 42 to 140 hours. The yeast culture can remain in the same container and use the same set of electromagnetic wave generator and emitters when switching from one EM field to another EM field.

The culture process can be initiated by inoculating 1000ml of medium with an inoculum of the selected yeast strain(s) of about 10⁸ cells. The starting culture is kept at 33±5 °C for 24 to 56 hours prior to exposure to the EM field(s). The culturing process may preferably be conducted under conditions in which the concentration of dissolved oxygen is between 0.025 to 0.08 mol/m³, preferably 0.04 mol/m³. The oxygen level can be controlled by any conventional means known in the art, including but not limited to stirring and/or bubbling. The culture is most preferably carried out in a liquid medium which contains animal serum and sources of nutrients assimilable by the yeast cells. Table 1 provides an exemplary medium for culturing the first yeast cell component of the invention.

**Table 1**

| Medium Composition | Quantity |
|---|---|
| Mannitol | 18.0g |
| K₂HPO₄ | 0.3g |
| MgSO₄•7H₂O | 0.2g |
| NaCl | 0.25g |
| CaSO₄•2H₂O | 0.2g |
| CaCO₃•5H₂O | 3.0g |
| Peptone | 1.2g |
| Bovine serum | 300ml |
| Autoclaved water | 700ml |

In general, carbohydrates such as sugars, for example, sucrose, glucose, fructose, dextrose, maltose, xylose, and the like and starches, can be used either alone or in combination as sources of assimilable carbon in the culture medium. The exact quantity of the carbohydrate source or sources utilized in the medium depends in part upon the other ingredients of the medium but, in general, the amount of carbohydrate usually varies between about 0.1% and 5% by weight of the medium and preferably between about 0.5% and 2%, and most preferably about 0.8%. These carbon sources can be used individually, or several such carbon sources may be combined in the medium. Among the inorganic salts which can be incorporated in the culture media are the customary salts capable of yielding sodium, calcium, phosphate, sulfate, carbonate, and like ions. Non-limiting examples of nutrient inorganic salts are (NH₄)₂HPO₄, CaCO₃, MgSO₄, NaCl and CaSO₄.

The bovine serum is a fraction of blood that comprises white blood cell, and can be prepared from whole blood (1000-2000 ml) by standard methods known in the art, such as density gradient centrifugation. Red blood cells are separated and discarded. The serum may be diluted or concentrated. The serum is added to the culture medium after the medium has been autoclaved and cooled to about 45 °C.

It should be noted that the composition of the media provided in Table 1 is not intended to be limiting. The process can be scaled up or down according to needs. Various modifications of the culture medium may be made by those skilled in the art, in view of practical and economic considerations, such as the scale of culture and local supply of media components.

Although the yeast cells will become activated even after a few hours of culturing in the presence of the EM field(s), the yeast cells can be cultured in the presence of the EM field(s) for an extended period of time (e.g., one or more weeks). At the end of the culturing process, the yeast cells which constitute the first yeast cell component of the invention may be recovered from the culture by various methods known in the art, and stored at a temperature below about 0°C to 4°C. The recovered yeast cells may also be dried and stored in powder form.

A non-limiting example of making a first yeast cell component of the invention with *Saccharomyces carlsbergensis* strain AS2.420 is provided in Section 6 hereinbelow. *Saccharomyces cerevisiae* strain AS2.562 is also preferred for making the first yeast cell component.

In another embodiment, the yeast cells of the second yeast cell component are cultured in the presence of at least one alternating electromagnetic (EM) field with a frequency in the range of 7000 MHz to 7030 MHz, preferably 7000-7020 MHz. A single EM field or a series of EM fields can be applied, each having a different frequency within the stated range, or a different field strength within the stated range, or different frequency and field strength within the stated ranges. Although any practical number of EM fields can be used within a series, it is preferred that, the yeast culture be exposed to a total of 2, 3, 4, 5, 6, 7, 8, 9 or 10 different EM fields in a series. The EM field(s), which can be applied by any means known in the art, can each have a frequency of 7000, 7001, 7002, 7003, 7004, 7005, 7006, 7007, 7008, 7009, 7010, 7011, 7012, 7013, 7014, 7015, 7016, 7017, 7018, 7019, or 7020 MHz. The field strength of the EM field(s) is in the range of 55 to 260 mV/cm preferably at 197 ± 4.0 mV/cm. The yeast cells can be cultured in the EM fields for 48 to 136 hours. The yeast culture can remain in the same container and use the same set of electromagnetic wave generator and emitters when switching from one EM field to another EM field.

The culture process can be initiated by inoculating 1000ml of medium with an inoculum of the selected yeast strain(s) of about 10⁸ cells. The starting culture is kept at 33±5°C for 24 to 56 hours prior to exposure to the EM field(s). The culturing process may preferably be conducted under conditions in which the concentration of dissolved oxygen is between 0.025 to 0.08 mol/m³, preferably 0.04 mol/m³. The oxygen level can be controlled by any conventional means known in the art, including but not limited to stirring and/or bubbling.

The culture is most preferably carried out in a liquid medium which contains animal serum and sources of nutrients assimilable by the yeast cells. Table 2 provides an exemplary medium for culturing the second yeast cell component of the invention.

**Table 2**

| Medium Composition | Quantity |
|---|---|
| Mannitol | 18.0g |
| K₂HPO₄ | 0.25g |
| MgSO₄•7H₂O | 0.2g |
| NaCl | 0.30g |
| CaSO₄•2H₂O | 0.1g |
| CaCO₃•5H₂O | 0.5g |
| Yeast extract | 0.4g |
| Bovine serum | 350ml |
| Autoclaved water | 650ml |

In general, carbohydrates such as sugars, for example, sucrose, glucose, fructose, dextrose, maltose, xylose, and the like and starches, can be used either alone or in combination as sources of assimilable carbon in the culture medium. The exact quantity of the carbohydrate source or sources utilized in the medium depends in part upon the other ingredients of the medium but, in general, the amount of carbohydrate usually varies between about 0.1% and 5% by weight of the medium and preferably between about 0.5% and 2%, and most preferably about 0.8%. These carbon sources can be used individually, or several such carbon sources may be combined in the medium. Among the inorganic salts which can be incorporated in the culture media are the customary salts capable of yielding sodium, calcium, phosphate, sulfate, carbonate, and like ions. Non-limiting examples of nutrient inorganic salts are (NH₄)₂HPO₄, CaCO₃, MgSO₄, NaCl, and CaSO₄.

It should be noted that the composition of the media provided in Table 2 is not intended to be limiting. The process can be scaled up or down according to needs. Various modifications of the culture medium maybe made by those skilled in the art, in view of practical and economic considerations, such as the scale of culture and local supply of media components.

Although the yeast cells will become activated even after a few hours of culturing in the presence of the EM field(s), the yeast cells can be cultured in the presence of the EM field(s) for an extended period of time (e.g., one or more weeks). At the end of the culturing process, the yeast cells which constitute the second yeast cell component of the invention may be recovered from the culture by various methods known in the art, and stored at a temperature below about 0°C to 4°C. The recovered yeast cells may also be dried and stored in powder form.

A non-limiting example of making a second yeast cell component of the invention with *Saccharomyces carlsbergensis* strain AS2.444 is provided in Section 6 hereinbelow. *Saccharomyces cerevisiae* strain AS2.560 is also preferred for making the second yeast cell component.

In yet another embodiment, the yeast cells of the third yeast cell component are cultured in the presence of at least one alternating electromagnetic (EM) field with a frequency in the range of 8900 MHz to 8930 MHz. A single EM field or a series of EM fields can be applied, each having a different frequency within the stated range, or a different field strength within the stated range, or different frequency and field strength within the stated ranges. Although any practical number of EM fields can be used within a series, it is preferred that, the yeast culture be exposed to a total of 2, 3, 4, 5, 6, 7, 8, 9 or 10 different EM fields in a series. The EM field(s), which can be applied by any means known in the art, can each have a frequency of 8900, 8901, 8902, 8903, 8904, 8905, 8906, 8907, 8908, 8909, 8910, 8911, 8912, 8913, 8914, 8915, 8916, 8917, 8918, 8919, 8920, 8921, 8922, 8923, 8924, 8925, 8926, 8927, 8928, 8929, or 8930 MHz. The field strength of the EM field(s) is in the range of 35 to 230 mV/cm preferably at 183 ± 3.0 mV/cm. The yeast cells can be cultured in the EM fields for 36 to 122 hours. The yeast culture can remain in the same container and use the same set of electromagnetic wave generator and emitters when switching from one EM field to another EM field.

The culture process can be initiated by inoculating 1000ml of medium with an inoculum of the selected yeast strain(s) of about 10⁸ cells. The starting culture is kept at 33±5°C for 24 to 56 hours prior to exposure to the EM field(s). The culturing process may preferably be conducted under conditions in which the concentration of dissolved oxygen is between 0.025 to 0.08 mol/m³, preferably 0.04 mol/m³. The oxygen level can be controlled by any conventional means known in the art, including but not limited to stirring and/or bubbling.

The culture is most preferably carried out in a liquid medium which contains animal serum and sources of nutrients assimilable by the yeast cells. Table 3 provides an exemplary medium for culturing the third yeast cell component of the invention.

**Table 3**

| Medium Composition | Quantity |
|---|---|
| Mannitol | 18.0g |
| K₂HPO₄ | 0.3g |
| MgSO₄•7H₂O | 0.30g |
| NaCl | 0.30g |
| CaSO₄•2H₂O | 0.40g |
| CaCO₃•5H₂O | 4.0g |
| Peptone | 1.5g |
| Bovine serum | 300ml |
| Autoclaved water | 700ml |

In general, carbohydrates such as sugars, for example, sucrose, glucose, fructose, dextrose, maltose, xylose, and the like and starches, can be used either alone or in combination as sources of assimilable carbon in the culture medium. The exact quantity of the carbohydrate source or sources utilized in the medium depends in part upon the other ingredients of the medium but, in general, the amount of carbohydrate usually varies between about 0.1% and 5% by weight of the medium and preferably between about 0.5% and 2%, and most preferably about 0.8%. These carbon sources can be used individually, or several such carbon sources may be combined in the medium. Among the inorganic salts which can be incorporated in the culture media are the customary salts capable of yielding sodium, calcium, phosphate, sulfate, carbonate, and like ions. Non-limiting examples of nutrient inorganic salts are (NH₄)₂HPO₄, CaCO₃, MgSO₄, NaCl, and CaSO₄.

It should be noted that the composition of the media provided in Table 3 is not intended to be limiting. The process can be scaled up or down according to needs. Various modifications of the culture medium may be made by those skilled in the art, in view of practical and economic considerations, such as the scale of culture and local supply of media components.

Although the yeast cells will become activated even after a few hours of culturing in the presence of the EM field(s), the yeast cells can be cultured in the presence of the EM field(s) for an extended period of time (e.g., one or more weeks). At the end of the culturing process, the yeast cells which constitute the third yeast cell component of the invention may be recovered from the culture by various methods known in the art, and stored at a temperature below about 0°C to 4°C. The recovered yeast cells may also be dried and stored in powder form.

A non-limiting example of making a third yeast cell component of the invention with *Saccharomyces carlsbergensis* strain AS2.162 is provided in Section 6 hereinbelow. *Saccharomyces cerevisiae* strain AS2.502 is also preferred for making the third yeast cell component.

In yet another embodiment, the yeast cells of the fourth yeast cell component are cultured in the presence of at least one alternating electromagnetic (EM) field with a frequency in the range of 9420 MHz to 9450 MHz, and preferably 9420-9440 MHz. A single EM field or a series of EM fields can be applied, each having a different frequency within the stated range, or a different field strength within the stated range, or different frequency and field strength within the stated ranges. Although any practical number of EM fields can be used within a series, it is preferred that, the yeast culture be exposed to a total of 2, 3, 4, 5, 6, 7, 8, 9 or 10 different EM fields in a series. The EM field(s), which can be applied by any means known in the art, can each have a frequency of 9420, 9421, 9422, 9423, 9424, 9425, 9426, 9427, 9428, 9429, 9430, 9431, 9432, 9433, 9434, 9435, 9436, 9437, 9438, 9439, or 9440 MHz. The field strength of the EM field(s) is in the range of 62 to 265 mV/cm at preferably 233 ± 4.0 mV/cm. The yeast cells can be cultured in the EM fields for 38 to 112 hours. The yeast culture can remain in the same container and use the same set of electromagnetic wave generator and emitters when switching from one EM field to another EM field.

The culture process can be initiated by inoculating 1000ml of medium with an inoculum of the selected yeast strain(s) of about 10⁸ cells. The starting culture is kept at 33±5 °C for 24 to 56 hours prior to exposure to the EM field(s). The culturing process may preferably be conducted under conditions in which the concentration of dissolved oxygen is between 0.025 to 0.08 mol/m³, preferably 0.04 mol/m³. The oxygen level can be controlled by any conventional means known in the art, including but not limited to stirring and/or bubbling.

The culture is most preferably carried out in a liquid medium which contains animal serum and sources of nutrients assimilable by the yeast cells. Table 4 provides an exemplary medium for culturing the fourth yeast cell component of the invention.

**Table 4**

| Medium Composition | Quantity |
|---|---|
| Mannitol | 18.0g |
| K₂HPO₄ | 0.22g |
| MgSO₄•7H₂O | 0.2g |
| NaCl | 0.23g |
| CaSO₄•2H₂O | 0.23g |
| CaCO₃•5H₂O | 2.5g |
| Peptone | 1.5 g |
| Bovine serum | 200ml |
| Autoclaved water | 800ml |

In general, carbohydrates such as sugars, for example, sucrose, glucose, fructose, dextrose, maltose, xylose, and the like and starches, can be used either alone or in combination as sources of assimilable carbon in the culture medium. The exact quantity of the carbohydrate source or sources utilized in the medium depends in part upon the other ingredients of the medium but, in general, the amount of carbohydrate usually varies between about 0.1% and 5% by weight of the medium and preferably between about 0.5% and 2%, and most preferably about 0.8%. These carbon sources can be used individually, or several such carbon sources may be combined in the medium. Among the inorganic salts which can be incorporated in the culture media are the customary salts capable of yielding sodium, calcium, phosphate, sulfate, carbonate, and like ions. Non-limiting examples of nutrient inorganic salts are (NH₄)₂HPO₄, CaCO₃, MgSO₄, NaCl, and CaSO₄.

It should be noted that the composition of the media provided in Table 4 is not intended to be limiting. The process can be scaled up or down according to needs. Various modifications of the culture medium may be made by those skilled in the art, in view of practical and economic considerations, such as the scale of culture and local supply of media components.

Although the yeast cells will become activated even after a few hours of culturing in the presence of the EM field(s), the yeast cells can be cultured in the presence of the EM field(s) for an extended period of time (e.g., one or more weeks). At the end of the culturing process, the yeast cells which constitute the fourth yeast cell component of the invention may be recovered from the culture by various methods known in the art, and stored at a temperature below about 0°C to 4°C. The recovered yeast cells may also be dried and stored in powder form.

A non-limiting example of making a fourth yeast cell component of the invention with *Saccharomyces carlsbergensis* strain AS2.559 is provided in Section 6 hereinbelow. Another *S. carlsbergensis* strain AS2.11 is also preferred for making the fourth yeast cell component.

### 5.2. CONDITIONING OF THE YEAST CELLS

According to the invention, performance of the activated yeast cells can be optimized by culturing the activated yeast cells in the presence of an extract from the stomach (e.g., the gastric juice) of a cattle. The inclusion of this additional conditioning or acclimatizing process allows the activated yeast cells to adapt to and endure the acidic environment of the subject's stomach. The method for conditioning or acclimatizing activated yeast cells of the invention comprises culturing yeast cells in the presence of gastric juice of an animal in an alternating electromagnetic (EM) field.

The culture process can be initiated by inoculating 1000ml of a conditioning medium with about 10 ml of activated yeasts containing about 10⁸ cells/ml (as prepared by the methods described in section 5.1). The initial number of yeast cells in the conditioning medium is about 10⁶ cells/ml. A equivalent number of dried yeast cells can also be used as an inoculum. The conditioning medium comprises per 1000 ml about 700 ml of gastric juice of an animal and about 300 ml of wild hawthorn juice. The process can be scaled up or down according to needs.

The gastric juice of a cattle can be obtained from the stomach content of a freshly slaughtered animal. Although not essential, the animal is preferably kept under a clean environment, and fed a standard diet, preferably germ-free. The gastric juice can be separated by centrifugation or filtration of the stomach content to remove debris and/or microorganisms. The gastric juice so obtained can be stored at 4°C. Preferably, the collection procedures and storage are carried out under sterile conditions.

The wild hawthorn juice is an extract of wild hawthorn fruits prepared by drying the fruits to less than about 8% moisture, crushing the dried fruits to less than about 20 mesh, and mixing 1500 ml of water per 500 gram of the crushed wild hawthorn. The mixture is then allowed to settle without stirring for 6 hours, and the clear liquid above is collected for use as the wild hawthorn juice used in the conditioning process. Other methods that can be used to collect the hawthorn juice include centrifugation or filtration of the mixture. Preferably, the collection procedures and storage are carried out under sterile conditions.

In one embodiment, the activated yeast cells of the first yeast cell component are cultured in conditioning medium in the presence of a series of two EM fields. The frequencies of the EM fields are 7713 and 7718 MHz. The field strength is in the range of 162 to 197 mV/cm, preferably at 162 ± 2 mV/cm. About 10⁹ activated yeast cells of the first yeast cell component are added to 1000 ml of conditioning medium. The temperature is maintained at 36 ± 2°C. The yeast culture is exposed to the EM field for about 42-140 hours.

In another embodiment, the activated yeast cells of the second yeast cell component are cultured in conditioning medium in the presence of a series of two EM fields. The frequencies of the EM fields are 7013 and 7018 MHz. The field strength is in the range of 162 to 183 mV/cm, preferably at 162 ± 2 mV/cm. About 10⁹ activated yeast cells of the second yeast cell component are added to 1000 ml of conditioning medium. The temperature is maintained at 36 ± 2°C. The yeast culture is exposed to the EM field for about 48-136 hours.

In yet another embodiment, the activated yeast cells of the third yeast cell component are cultured in conditioning medium in the presence of a series of two EM fields. The frequencies of the EM fields are 8906 and 8910 MHz. The field strength is in the range of 162 to 183 mV/cm, preferably at 162 ± 2 mV/cm. About 10⁹ activated yeast cells of the third cell component are added to 1000 ml of conditioning medium. The temperature is maintained at 36 ± 2°C. The yeast culture is exposed to the EM field for about 36-122 hours.

In yet another embodiment, the activated yeast cells of the fourth yeast cell component are cultured in conditioning medium in the presence of a series of two EM fields. The frequencies of the EM fields are 9431 and 9437 MHz. The field strength is in the range of 50 to 250 mV/cm, preferably at 233 ± 2 mV/cm. About 10⁹ activated yeast cells of the first yeast cell component are added to 1000 ml of conditioning medium. The temperature is maintained at 36 ± 2°C. The yeast culture is exposed to the EM field for about 38-112 hours.

The activated and conditioned yeast cells of all four yeast cell components may be recovered from the culture by various methods known in the art, and preferably stored in powder form at a temperature below about 0°C to 4°C. Preferably, the powder form of the yeast cells comprises greater than about 10¹⁰ yeast cells per gram. The activated and conditioned yeast cells can be dried as follows: the yeast cells was dried in a first dryer at a temperature not exceeding 60 ± 2°C for a period of 5 minutes so that yeast cells quickly became dormant. The yeast cells were then sent to a second dryer and dried at a temperature not exceeding 65 ± 2°C for a period of about 8 minutes to further remove water. The dried yeast cells were then cool to room temperature.

The activated and conditioned yeast cells can be used separately but preferably as a mixture in a biological composition, or used as a starter culture for large scale manufacturing.

### 5.3 MANUFACTURE OF THE BIOLOGICAL COMPOSITIONS

The present invention also encompasses methods of manufacturing of the biological compositions of the invention. The activated and conditioned yeast cells as prepared by section 5.1 and 5.2 can be propagated on a large scale to make the biological compositions of the invention. The method comprises culturing the yeast cells of the four yeast cell components separately in the presence of one or more EM fields for a period of time, diluting the growing yeast cells with fresh medium, and repeating the process. The method can be carried out as a batch process or a continuous process.

In one preferred embodiment, a set of three containers each comprising a set of electrodes for generating an electromagnetic field as described in section 5.1 and 5.2 are set up each with 1000 liters of a culture medium. The culture medium comprises nutrients assimilable by the yeast cells as shown in Table 5.

**Table 5**

| **Material** | **Quantity** |
|---|---|
| Wild hawthorn juice | 200 liters |
| Starch. | 30 kg |
| peptone | 5 kg |
| Distilled water | 800 liters |

The wild hawthorn juice is an extract of fresh wild hawthorn fruits prepared by washing the fruits clean, drying the fruits to less than about 8% moisture, crushing the dried fruits to less than about 20 mesh, and mixing the crushed wild hawthorn with water at a ratio of 1 liters of water per 100 gram of crushed fruits. The mixture is then stirred continuously for 12 hours while the temperature is maintained at 28°C to 30°C. The mixture is then centrifuged at 1000 rpm to collect the supernatant which is used as described above. Preferably, the procedures are carried out under sterile conditions.

The first container(s) is inoculated with activated and conditioned yeast cells. The yeast cells in the first container is used as a seed culture to inoculate the culture medium in second container. About 5 ml of yeast cells (1 x 10⁸ cells/ml) in the first container is added per 100 ml of culture medium in the second container.

The yeast cells in the second container (6) are then subjected to one or more EM fields. For the activated and conditioned yeast cells of the first yeast cell component, the frequencies of 7703, 7708, 7713 or 7718 MHz. The field strength is in the range of 30 to 230 mV/cm. The temperature is maintained at 36 ± 1°C. The yeast culture is exposed to the EM field for about 42-140 hours.

For the activated and conditioned yeast cells of the second yeast cell component, the frequency of the EM field is 7003, 7007, 7013 or 7018 MHz are used. The field strength is in the range of 30 to 230 mV/cm. The temperature is maintained at 36 ± 2°C. The yeast culture is exposed to the EM field for about 48-130 hours.

For the activated and conditioned yeast cells of the third yeast cell component, EM fields of frequencies of 8901, 8906, 8910 or 8916 MHz are used. The field strength is in the range of 30 to 230 mV/cm. The temperature is maintained at 36 ± 1°C. The yeast culture is exposed to the EM field for about 36-122 hours.

For the activated and conditioned yeast cells of the fourth yeast cell component, EM fields of frequencies of 9421, 9427, 9431 or 9437 MHz are used. The field strength is in the range of 30 to 230 mV/cm. The temperature is maintained at 36 ± 2°C. The yeast culture is exposed to the EM field for about 38-112 hours.

After culturing in the second container, the yeast cells are transferred into the third container and further cultured in the presence of EM fields using the same set of EM field characteristics described above for culturing in the second container.

The yeast cell culture resulting from the end of this stage can be used directly as a biological composition of the invention, or mixed in equal proportions to form a preferred biological composition that comprises all four yeast cell components.

Preferably, a biological feed additive comprising all four yeast cell components is prepared by adsorbing the yeast cells to an absorbent carrier. To facilitate this, the yeast cell culture is concentrated using methods known in the art. The concentration process is carried out in two stages. At the first stage, the volume of the liquid culture is reduced to about 80% of the original volume. At the end of the second stage, the volume is reduced to about 50%. The biological feed additive comprising all four yeast cell components can be prepared by mixing yeast cells of each component with an absorbent carrier such as starch or zeolite powder (less than 200 mesh) at a ratio of 100 to 120 liters of concentrated yeast cells (5-10 kg dried cells) per 1000 kg of feed additive. For every 990 to 995 kg of carrier, 5 to 10 kg of the yeast and zeolite powder mixture was added. The mixture is dried at a temperature not exceeding 65 °C for a period of time less than 10 minutes such that the yeast cells become dormant, and the moisture content is below 5%. The final dried product comprises at least about 10⁵ yeasts per gram and preferably greater than or equal to about 10⁸ yeasts per gram. The final dried product can be used by mixing it with animal feed at a ratio of about 0.2 to 0.6%.

### 6. EXAMPLE

The following example illustrates the manufacture of a biological composition that can be used as an animal feed additive.

The biological composition comprises the following four components of yeasts: *Saccharomyces carlsbergensis* AS2.420, AS2.444, *Saccharomyces cerevisiae* AS2.502 and AS2.559. Each component of the yeast cells is capable of inhibiting the growth of E. coli 0157:H7 cells and reducing the number of E. coli 0157:H7 cells in culture. The four yeast cell components are prepared and tested separately as follows:

A starting culture containing about 10⁵ cells/ml of AS2.420 is placed into the container (2) as shown in Figure 1. The medium has the composition shown in Table 1. Initially, the yeast cells are cultured for about 24-56 hours at 33 ± 5°C without an EM field. Then, in the same medium, at 33 ± 5 °C, the yeast cells are cultured in the presence of a series of four EM fields applied in the order stated: 7703MHz at 156mv/cm for 10 hrs; 7708MHz at 156mv/cm for 10 hrs; 7713MHz at 156mv/cm for 60 hrs; and 7718MHz at 156mv/cm for 60 hrs. The yeast cells were conditioned by further culturing in the gastric juice of a cattle and hawthorn juice as described in section 5.2, in the presence of a series of two EM fields: 7713 MHz at 156 mV/cm for 60 hours and 7718 MHz at 156 mV/cm for 60 hours. After the last culture period, the yeast cells are either used within 24 hours to make the biological compositions, or dried for storage as described in section 5.3.

The activated and conditioned yeast cells of the first yeast cell component was tested for their effect on *E*. *coli* O157:H7 cells in vitro. 300 ml of bovine serum was obtained from a cattle (of a Dutch breed kept for meat) under sterile conditions. 0.15 ml of activated and conditioned AS 2.420 yeast cells (10⁹ cells per ml) were added to the bovine serum and the mixture was kept in a tissue culture incubator at 37 °C under CO₂ for about 12 hours. One ml of *E. coli* 0157:H7 cells (10¹² per ml) was added to the bovine serum-yeast cell mixture which was incubated for another 12 hours. The number of *E. coli* O157:H cells remaining in culture was counted. The experiment was repeated with a blank (i.e, no yeast cells were added), and with AS2.420 yeast cells which were neither activated nor conditioned. The results are shown in Table 6 below.

**Table 6:**

| Experimental Groups | Concentration of E. coli after 12 hours of incubation | Concentration of E. coli before incubation | Percentage change in concentration of E. coli |
|---|---|---|---|
| Activated and Conditioned AS2.420 | 0.1 x 10⁹ /ml | 3 x 10⁹ /ml | 3.3% |
| Non-activated non-conditioned AS2.420 | 12 x 10⁹ /ml | 3 x 10⁹ /ml | 400% |
| No yeast cells | 15 x 10⁹ /ml | 3 x 10⁹ /ml | 500% |

To prepare the second component, a starting culture containing about 10⁵ cells/ml of AS2.444 is placed into the container (2) as shown in Figure 1. The medium has a composition as shown in Table 2. Initially, the yeast cells are cultured for about 24 to 56 hours at 33 ± 5 °C without an EM field. Then, in the same medium, at 33 ± 5 °C, the yeast cells are cultured in the presence of a series of four EM fields applied in the order stated: 7003MHz at 197mv/cm for 10 hrs; 7007MHz at 197mv/cm for 10 hrs; 7013MHz at 197mv/cm for 58 hrs; and 7018MHz at 197 mv/cm for 58 hrs. The yeast cells were conditioned by further culturing in the gastric juice of a cattle and hawthorn juice as described in section 5.2, in the presence of a series of two EM fields: 7013 MHz at 197 mV/cm for 58 hours and 7018 MHz at 197 mV/cm for 58 hours. After the last culture period, the yeast cells are either used within 24 hours to make the biological compositions, or dried for storage as described in section 5.3.

The activated and conditioned yeast cells of the second yeast cell component was tested for their effect on *E. coli* O157:H cells in vitro. 300 ml of bovine serum was obtained from a cattle (of a Dutch breed kept for meat) under sterile conditions. 0.15 ml of activated and conditioned AS 2.444 yeast cells (10⁹ cells per ml) were added to the bovine serum and the mixture was kept in a tissue culture incubator at 37°C under CO₂ for about 12 hours. One ml of *E. coli* O157:H7 cells (10¹² per ml) was added to the bovine serum-yeast cell mixture which was incubated for another 12 hours. The number of *E. coli* 0157:H7 cells remaining in culture was counted. The experiment was repeated with a blank (i.e, no yeast cells were added), and with AS2.444 yeast cells which were neither activated nor conditioned. The results are shown in Table 7 below.

**Table 7:**

| Experimental Groups | Concentration of E. coli after 12 hours of incubation | Concentration of E. coli before incubation | Percentage change in concentration of E. coli |
|---|---|---|---|
| Activated and Conditioned AS2.444 | 0.15 x 10⁹ /ml | 3 x 10⁹ /ml | 5% |
| Non-activated non-conditioned AS2.420 | 11 x 10⁹ /ml | 3 x10⁹ /ml | 367% |
| No yeast cells | 15 x 10⁹ /ml | 3 x 10⁹ /ml | 500% |

For the third yeast cell component, a starting culture containing about 10⁵ cells/ml of AS2.502 is placed into the container (2) as shown in Figure 1. The medium has a composition as shown in Table 3. Initially, the yeast cells are cultured for about 42 hours at 33 ± 5 °C without an EM field. Then, in the same medium, at 33 ± 5 °C, the yeast cells are cultured in the presence of a series of four EM fields applied in the order stated: 8901MHz at 183mv/cm for 53 hrs; 8906MHz at 183mv/cm for 53 hrs; 8910MHz at 183mv/cm for 8 hrs; and 8916MHz at 183mv/cm for 8 hrs. The yeast cells were conditioned by further culturing in the gastric juice of a cattle and hawthorn juice as described in section 5.2, in the presence of a series of two EM fields: 8901 MHz at 183 mV/cm for 53 hours and 8906 MHz at 183 mV/cm for 53 hours. After the last culture period, the yeast cells are either used within 24 hours to make the biological compositions, or dried for storage as described in section 5.3.

The activated and conditioned yeast cells of the third yeast cell component was tested for their effect on *E*. *coli* 0157:H7 cells in vitro. 300 ml of bovine serum was obtained from a cattle (of a Dutch breed kept for meat) under sterile conditions. 0.15 ml of activated and conditioned AS 2.502 yeast cells (10⁹ cells per ml) were added to the bovine serum and the mixture was kept in a tissue culture incubator at 37 °C under CO₂ for about 12 hours. One ml of *E. coli* 0157:H7 cells (10¹² per ml) was added to the bovine serum-yeast cell mixture which was incubated for another 12 hours. The number of *E. coli* O157:H cells remaining in culture was counted. The experiment was repeated with a blank (i.e, no yeast cells were added), and with AS2.502 yeast cells which were neither activated nor conditioned. The results are shown in Table 8 below.

**Table 8:**

| Experimental Groups | Concentration of E. coli after 12 hours of incubation | Concentration of E. coli before incubation | Percentage change in concentration of E. coli |
|---|---|---|---|
| Activated and Conditioned AS2.502 | 0.23 x 10⁹ /ml | 3 x 10⁹ /ml | 7.7% |
| Non-activated non-conditioned AS2.502 | 13 x 10⁹ /ml | 3 x 10⁹ /ml | 433% |
| No yeast cells | 15 x 10⁹ /ml | 3 x 10⁹ /ml | 500% |

To prepare the fourth component, a starting culture containing about 10⁵ cells/ml of AS2.559 is placed into the container (2) as shown in Figure 1. The medium has a composition as shown in Table 4. Initially, the yeast cells are cultured for about 52 hours at 33 ± 5°C without an EM field. Then, in the same medium, at 33 ± 5 °C, the yeast cells are cultured in the presence of a series of four EM fields applied in the order stated: 9421MHz at 233mv/cm for 10 hrs; 9427MHz at 233mv/cm for 10 hrs; 9431MHz at 233mv/cm for 46 hrs; and 9437MHz at 233mv/cm for 46 hrs. The yeast cells were conditioned by further culturing in the gastric juice of a cattle and hawthorn juice as described in section 5.2, in the presence of a series of two EM fields: 9431 MHz at 233 mV/cm for 46 hours and 9437 MHz at 233 mV/cm for 46 hours. After the last culture period, the yeast cells are either used within 24 hours to make the biological compositions, or dried for storage as described in section 5.3.

The activated and conditioned yeast cells of the fourth yeast cell component was tested for their effect on *E*. *coli* O157:H cells in vitro. 300 ml of bovine serum was obtained from a cattle (of a Dutch breed kept for meat) under sterile conditions. 0.15 ml of activated and conditioned AS 2.559 yeast cells (10⁹ cells per ml) were added to the bovine serum and the mixture was kept in a tissue culture incubator at 37°C under CO₂ for about 12 hours. One ml of *E*. *coli* O157:H7 cells (10¹² per ml) was added to the bovine serum-yeast cell mixture which was incubated for another 12 hours. The number of *E*. *coli* 0157:H7 cells remaining in culture was counted. The experiment was repeated with a blank (i.e, no yeast cells were added), and with AS2.559 yeast cells which were neither activated nor conditioned. The results are shown in Table 9 below.

**Table 9:**

| Experimental Groups | Concentration of E. coli after 12 hours of incubation | Concentration of E. coli before incubation | Percentage change in concentration of E. coli |
|---|---|---|---|
| Activated and Conditioned AS2.559 | 0.23 x 10⁹ /ml | 3 x 10⁹ /ml | 7.7% |
| Non-activated non-conditioned AS2.559 | 10.5 x 10⁹ /ml | 3 x 10⁹ /ml | 350% |
| No yeast cells | 15 x 10⁹ /ml | 3 x 10⁹ /ml | 500% |

The activated and conditioned yeast cells of all four yeast cell components were tested together for their effect on *E*. *coli* O157:H7 cells in vitro. 300 ml of bovine serum was obtained from a cattle (of a Dutch breed kept for meat) under sterile conditions. 0.15 ml of activated and conditioned yeast cells (10⁹ cells per ml) from each of the four yeast cell components as described above (AS2.420, AS2.444, AS2.502 and AS2.559) were added to the bovine serum and the mixture was kept in a tissue culture incubator at 37°C under CO₂ for about 12 hours. One ml of *E*. *coli* 0157:H7 cells (10¹² per ml) was added to the bovine serum - yeast cell mixture which was incubated for another 12 hours. The number of *E*. *coli* O157:H7 cells remaining in culture was counted. The experiment was repeated with a blank (i.e, no yeast cells were added), and with AS2.420 yeast cells which were neither activated nor conditioned. The results are shown in Table 10 below.

**Table 10:**

| Experimental Groups | Concentration of E. coli after 12 hours of incubation | Concentration of E. coli before incubation | Percentage change in concentration of E. coli |
|---|---|---|---|
| Activated and Conditioned yeast cells | undetectable | 3 x 10⁹ /ml | n.a. |
| Non-activated non-conditioned yeast cells | 9.6 x 10⁹ /ml | 3 x 10⁹ /ml | 320% |
| No yeast cells | 15 x 10⁹ /ml | 3 x 10⁹ /ml | 500% |

The present invention is not to be limited in scope by the specific embodiments described which are intended as single illustrations of individual aspects of the invention, and functionally equivalent methods and components are within the scope of the invention. Indeed various modifications of the invention, in addition to those shown and described herein will become apparent to those skilled in the art from the foregoing description and accompanying drawings. Such modifications are intended to fall within the scope of the appended claims.

## Claims

1. A biological composition comprising at least one of the following yeast cell components:
(a) a first yeast cell component comprising a plurality of yeast cells that are prepared by culturing the yeast cells in an electromagnetic field or a series of electromagnetic fields having a frequency in the range of 7700 to 7722 MHz and a field strength of 20 to 220 mV/cm;
(b) a second yeast cell component comprising a plurality of yeast cells that are prepared by culturing the yeast cells in an electromagnetic field or a series of electromagnetic fields having a frequency in the range of 7000 to 7020 MHz and a field strength of 55 to 260 mV/cm;
(c) a third yeast cell component comprising a plurality of yeast cells that are prepared by culturing the yeast cells in an electromagnetic field or a series of electromagnetic fields having a frequency in the range of 8900 to 8930 MHz and a field strength of 35 to 230 mV/cm; and
(d) a fourth yeast cell component comprising a plurality of yeast cells that are prepared by culturing the yeast cells in an electromagnetic field or a series of electromagnetic fields having a frequency in the range of 9420 to 9440 MHz and a field strength of 62 to 265 mV/cm.

2. The biological composition of claim 1 which comprises the yeast cell components of (a), (b), (c) and (d).

3. The biological composition of claim 1 or 2, wherein the yeast cells are cells of Saccharomyces.

4. The biological composition of claim 1 or 2, wherein the yeast cells are cells of Saccharomyces cerevisiae or Saccharomyces carlsbergensis.

5. The biological composition of claim 1 or 2 in which the yeast cells are dried.

6. An animal feed composition comprising the biological composition of claim 1 or 2, and cattle feed.

7. The animal feed composition of claim 6 wherein the biological composition further comprises zeolite powder at a ratio of at least 10⁹ yeast cells to 1 g of zeolite powder.

8. The animal feed composition of claim 7 in which at least 0.5% by weight is the biological composition of claim 1 or 2.

9. A method for preparing a biological composition, said method comprising culturing a plurality of yeast cells in an electromagnetic field or a series of electromagnetic fields having a frequency in the range of 7700 to 7722 MHz and a field strength of 20 to 220 mV/cm.

10. The method of claim 9, wherein said method further comprises culturing the plurality of yeast cells in one or more of the electromagnetic fields in a culture medium comprising gastric juice of cattle and wild hawthorn juice.

11. A method for preparing a biological composition, said method comprising culturing a plurality of yeast cells in an electromagnetic field or a series of electromagnetic fields having a frequency in the range of 7000 to 7020 MHz and a field strength of 55 to 260 mV/cm.

12. The method of claim 11, wherein said method further comprises culturing the plurality of yeast cells in one or more of the electromagnetic fields in a culture medium comprising gastric juice of cattle and wild hawthorn juice.

13. A method for preparing a biological composition, said method comprising culturing a plurality of yeast cells in an electromagnetic field or a series of electromagnetic fields having a frequency in the range of 8900 to 8930 MHz and a field strength of 35 to 230 mV/cm.

14. The method of claim 13, wherein said method further comprises culturing the plurality of yeast cells in one or more of the electromagnetic fields in a culture medium comprising gastric juice of cattle and wild hawthorn juice.

15. A method for preparing a biological composition, said method comprising culturing a plurality of yeast cells in an electromagnetic field or a series of electromagnetic fields having a frequency in the range of 9420 to 9440 MHz and a field strength of 62 to 265 mV/cm.

16. The method of claim 15, wherein said method further comprises culturing the plurality of yeast cells in one or more of the electromagnetic fields in a culture medium comprising gastric juice of cattle and wild hawthorn juice.

17. A method of making an animal feed composition, said method comprising
(a) preparing one or more of the yeast cell components of claim 1,
(b) adsorbing the yeast cell components of (a) to a carrier, and
(c) mixing the dried yeast cells and carrier with cattle feed.

18. The method of claim 17, wherein the adsorbing step comprises (i) concentrating the yeast cell culture by about 50%, (ii) mixing the yeast cell culture with the carrier; and (iii) drying the mixture at a temperature not exceeding 65 °C to reduce the moisture content to below 5%.

19. A method for reducing the incidence of infectious diseases by a pathogenic strain of Escherichia coli in a cattle comprising feeding the cattle for a period of time an animal feed composition comprising at least one of the following yeast cell components:
(a) a first yeast cell component comprising a plurality of yeast cells that are prepared by culturing the yeast cells in an electromagnetic field or a series of electromagnetic fields having a frequency in the range of 7700 to 7722 MHz and a field strength of 20 to 220 mV/cm;
(b) a second yeast cell component comprising a plurality of yeast cells that are prepared by culturing the yeast cells in an electromagnetic field or a series of electromagnetic fields having a frequency in the range of 7000 to 7020 MHz and a field strength of 55 to 260 mV/cm;
(c) a third yeast cell component comprising a plurality of yeast cells that are prepared by culturing the yeast cells in an electromagnetic field or a series of electromagnetic fields having a frequency in the range of 8900 to 8930 MHz and a field strength of 35 to 230 mV/cm; and
(d) a fourth yeast cell component comprising a plurality of yeast cells that are prepared by culturing the yeast cells in an electromagnetic field or a series of electromagnetic fields having a frequency in the range of 9420 to 9440 MHz and a field strength of 62 to 265 mV/cm.

20. The method of claim 19, wherein the animal feed composition comprises the yeast cell components of (a), (b), (c) and (d), and zeolite powder.

21. The method of claim 19, wherein said yeast cells are *Saccharomyces carlsbergensis* or *Saccharomyces cerevisiae* cells.

22. The method of claim 19, wherein the yeast cell components and zeolite powder comprises 0.5% by weight of the animal feed composition.

23. The composition of claim 1 or 2, wherein the plurality of yeast cells used in preparing the first yeast cell component comprise cells of *Saccharomyces carlsbergensis* AS2.420, wherein the plurality of yeast cells used in preparing the second yeast cell component comprise cells *of Saccharomyces carlsbergensis* AS2.444, wherein the plurality of yeast cells used in preparing the third yeast cell component comprise cells of *Saccharomyces cerevisiae* AS2.502, and wherein the plurality of yeast cells used in preparing the fourth yeast cell component comprise cells of *Saccharomyces cerevisiae* AS2.559.

24. The animal feed composition of claim 6, wherein the plurality of yeast cells used in preparing the first yeast cell component comprise cells *of Saccharomyces carlsbergensis* AS2.420, wherein the plurality of yeast cells used in preparing the second yeast cell component comprise cells of *Saccharomyces carlsbergensis* AS2.444, wherein the plurality of yeast cells used in preparing the third yeast cell component comprise cells of *Saccharomyces cerevisiae* AS2.502, and wherein the plurality of yeast cells used in preparing the fourth yeast cell component comprise cells of *Saccharomyces cerevisiae* AS2.559.
